# EUROPEAN PATENT APPLICATION

(11) **EP 4 502 573 A1**
(43) Date of publication of application: **05.02.2025**
(21) Application number: 23780543.7
(22) Date of filing: 28.03.2023
(51) Int. Cl.: G01N 15/02, G01N 21/64, C12M 1/00, C12M 1/34

(54) **PARTICLE ANALYSIS DEVICE, PARTICLE ANALYSIS DEVICE PROGRAM, AND PARTICLE ANALYSIS METHOD**

(30) Priority: 31.03.2022 JP 2022058318
(71) Applicant: HORIBA, Ltd., Kyoto-shi Kyoto 601-8510 (JP)
(72) Inventor: SUGASAWA, Hirosuke, Kyoto-shi, Kyoto 601-8510 (JP); TATEWAKI, Yasuhiro, Kyoto-shi, Kyoto 601-8510 (JP); IGUSHI, Tatsuo, Kyoto-shi, Kyoto 601-8510 (JP)
(74) Representative: Isarpatent
(86) International application number: PCT/JP2023/012555
(87) International publication number: WO 2023/190534

(57) **Abstract**

In order to make it possible to secure sufficient observation time in the PTA method without being affected by fading of a fluorescent marker or a particle having the property of autofluorescence, a particle analysis device in accordance with this invention is a particle analysis device 100 that can take a fluorescence observation mode in which fluorescence emitted by a fluorescent marker added to a particle or by the particle itself is imaged by irradiating the particle with excitation light, and a scattered light observation mode in which scattered light produced by irradiating the particle with the light is imaged, and is characterized by comprising a particle specifying unit 41 that specifies the particle to which the fluorescent marker is added or the particle that emits the fluorescence from fluorescence image data obtained in the fluorescence observation mode, and an analyzing unit 42 that analyzes physical properties of the particle by obtaining diffusion speed due to the Brownian motion of the particle specified by the particle specifying unit 41 from the image data of the scattered light obtained in the scattered light observation mode having a frame number larger than that in the fluorescence observation mode.

## Description

### Technical field

This invention relates to a particle analysis device that analyzes a particle by the particle trajectory analysis method (PTA method).

### Background Art

As shown in the Patent Document 1, this type of the particle analysis device analyzes the physical properties of a particle by adding a fluorescent marker to the particle as an object to be measured, irradiating the particle with a laser beam having an excitation wavelength corresponding to the fluorescent marker, and observing the fluorescence produced by irradiating the laser beam.

Concretely, the particle analysis device comprises a filter that cuts off the laser light scattered by the particle while transmitting the fluorescence emitted by the fluorescent marker and is so configured that it is possible to conduct analysis while separating the particle to which the fluorescent marker is added from other particle by observing the fluorescence transmitted through the filter by an imaging unit.

When the fluorescent marker is irradiated with intense light such as laser light, a phenomenon as so-called fading wherein the fluorescent dye is photochemically destroyed and no longer generates the fluorescence.

In the above-mentioned PTA method, since particle diameter is calculated from an average travelling distance of the particle within a certain period of time, it is necessary to observe the particle for a certain period of time. However, there is a case that it is not possible to secure a sufficient period of time to make observation if the above-mentioned fading occurs.

Although some countermeasures against fading can be expected to improve the fading to some extent such as reducing the intensity of the excitation light irradiating on the fluorescent marker, reducing the frame rate by lengthening the exposure time, and using fluorescent dye that is resistant to fading, there are limits to how much these improvements can be made.

The above-mentioned problems are not limited to a case of analyzing the particle to which the fluorescent marker is added but can also commonly occur when analyzing a particle having auto-fluorescent properties.

### Prior art documents

### Patent document

Patent Document 1: Japan Unexamined Patent Application Publication No. 2020-204604

### Summary of the Invention

### Problems to be solved by the invention

The present invention is to solve the above-mentioned problem and is intended to secure sufficient observation time in the PTA method without being affected by fading of a fluorescent marker or a particle having the property of auto-fluorescence.

More specifically, a particle analysis device in accordance with the present claimed invention is a particle analysis device that can take a fluorescence observation mode in which fluorescence emitted by a fluorescent marker added to a particle or by the particle itself is imaged by irradiating the particle with excitation light, and a scattered light observation mode in which scattered light produced by irradiating the particle with the light is imaged, and is characterized by comprising a particle specifying unit that specifies the particle to which the fluorescent marker is added or the particle that emits the fluorescence from fluorescence image data obtained in the fluorescence observation mode, and an analyzing unit that analyzes physical properties of the particle by obtaining diffusion speed due to the Brownian motion of the particle specified by the particle specifying unit from the image data of the scattered light obtained in the scattered light observation mode having a frame number larger than that in the fluorescence observation mode.

In accordance with the particle analysis device having the above configuration, since the particle to which the fluorescent marker is added or the particle that emits the fluorescence is specified in the fluorescence observation mode, and the specified particle is observed and analyzed in the scattered light observation mode, it is possible to reduce the time required for the fluorescence observation mode to a short period, and it is possible to secure a sufficient observation time by the PTA method without being affected by the fading of the color of the fluorescent marker in the subsequent scattered light observation mode.

It is preferable to further comprise a filter that cuts the scattered light produced by irradiating the particle with the excitation light while transmitting the fluorescence emitted by the fluorescent marker or the particle itself, and an imaging unit that images the fluorescence transmitted through the filter.

In accordance with this configuration, it is possible to image the fluorescence without being affected by the scattered light generated by the irradiation of the excitation light to the particle, and to more reliably specify the particle to which the fluorescent marker is added or the particle emitting the fluorescence.

It is preferable that the filter is arranged in front of the imaging unit in the fluorescence observation mode, and the filter is removed from in front of the imaging unit in the scattered light observation mode.

As mentioned above, it is possible to use a common imaging unit both in the fluorescence observation mode and in the scattered light observation mode by removing the filter from in front of the imaging unit in the scattered light observation mode, resulting in reducing a number of components and a manufacturing cost and downsizing the particle analysis device.

It is preferable to further comprise a mode switching mechanism that removes the filter that has been arranged in front of the imaging unit at a time of starting the fluorescence observation mode from in front of the imaging unit after a predetermined time elapses from the start of the fluorescence observation mode.

In accordance with this configuration, since the mode switching mechanism removes the filter from in front of the imaging unit, it enables mode switching, resulting in automating the analysis.

As another embodiment to enable both the fluorescence observation mode and the scattered light observation mode represented is the particle analysis device that further comprises a beam splitter that splits a luminous flux comprising the fluorescence and the scattered light into a first optical path and a second optical path, and is characterized by that the imaging unit and the filter are arranged on the first optical path, and a second imaging unit that is different from the imaging unit that observes the scattered light is arranged on the second optical path.

In accordance with this configuration, it is possible to install the filter on the first optical path and there is no need of providing a mechanism or an operation to remove the filter from in front of the imaging unit, and furthermore, it makes it possible to observe the fluorescence and the scattered light simultaneously.

It is preferable that the particle specifying unit specifies the particle to which the fluorescent marker is added or the particle that emits fluorescence from at least the first frame of the image data of the fluorescence obtained in the fluorescence observation mode.

In accordance with this configuration, it is possible to shorten time required as the fluorescence observation mode as much as possible.

It is preferable that at least one of intensity of the excitation light, a gain of the imaging unit, exposure time of the imaging unit and a position of the filter differs between the fluorescence observation mode and the scattered light observation mode.

In accordance with this configuration, it is possible to set an appropriate imaging condition for the fluorescence observation mode and the scattered light observation mode respectively.

A particle analysis device program in accordance with this invention is a program used for a particle analysis device that can take a fluorescence observation mode in which fluorescence emitted by a fluorescent marker added to a particle or by the particle itself is imaged by irradiating the particle with excitation light, and a scattered light observation mode in which scattered light produced by irradiating the particle with the light is imaged, and is characterized by making a computer produce functions as a particle specifying unit that specifies the particle to which the fluorescent marker is added or the particle that emits the fluorescence from fluorescence image data obtained in the fluorescence observation mode, and an analyzing unit that analyzes physical properties of the particle by obtaining diffusion speed due to the Brownian motion of the particle specified by the particle specifying unit from the image data of the scattered light obtained in the scattered light observation mode having a frame number larger than that in the fluorescence observation mode.

A particle analysis method in accordance with this invention is a particle analysis method for use in a particle analysis device that can take a fluorescence observation mode in which fluorescence emitted by a fluorescent marker added to a particle or by the particle itself is imaged by irradiating the particle with excitation light, and a scattered light observation mode in which scattered light produced by irradiating the particle with the light is imaged, and is characterized by that the particle to which the fluorescent marker is added or the particle that emits the fluorescence from fluorescence image data obtained in the fluorescence observation mode is specified, and the physical properties of the particle by obtaining diffusion speed due to the Brownian motion of the particle specified by the particle specifying unit from the image data of the scattered light obtained in the scattered light observation mode having a frame number larger than that in the fluorescence observation mode is analyzed.

In accordance with the particle analysis device program and the particle analysis method, the same effects as those of the above-mentioned particle analysis device can be achieved.

### Effects of the Invention

In accordance with the above-mentioned present claimed invention, it is possible to secure sufficient observation time by the PTA method without being affected by the fading of the fluorescent marker.

### Brief description of the drawings

[Fig. 1] A schematic diagram showing a fluorescence observation mode of a particle analysis device in accordance with one embodiment of this invention.
[Fig. 2] A schematic diagram showing a scattered light observation mode of the particle analysis device of this embodiment.
[Fig. 3] A table showing an example of a fluorescent marker used in this embodiment.
[Fig. 4] A functional block diagram showing functions of an information processing unit in this embodiment.
[Fig. 5] A flowchart showing operation of the particle analysis device in this embodiment.
[Fig. 6] A schematic diagram showing an image obtained by an imaging unit in this embodiment.
[Fig. 7] A schematic diagram showing a fluorescence observation mode of a particle analysis device in another embodiment.
[Fig. 8] A schematic diagram showing a scattered light observation mode of the particle analysis device in another embodiment.
[Fig. 9] A schematic diagram showing the particle analysis device in a further different embodiment.
[Fig. 10] A schematic diagram showing the particle analysis device in a further different embodiment.

### Best mode for embodying the invention

One embodiment of a particle analysis device of the present claimed invention will be described below with reference to drawings.

### <Device Configuration>

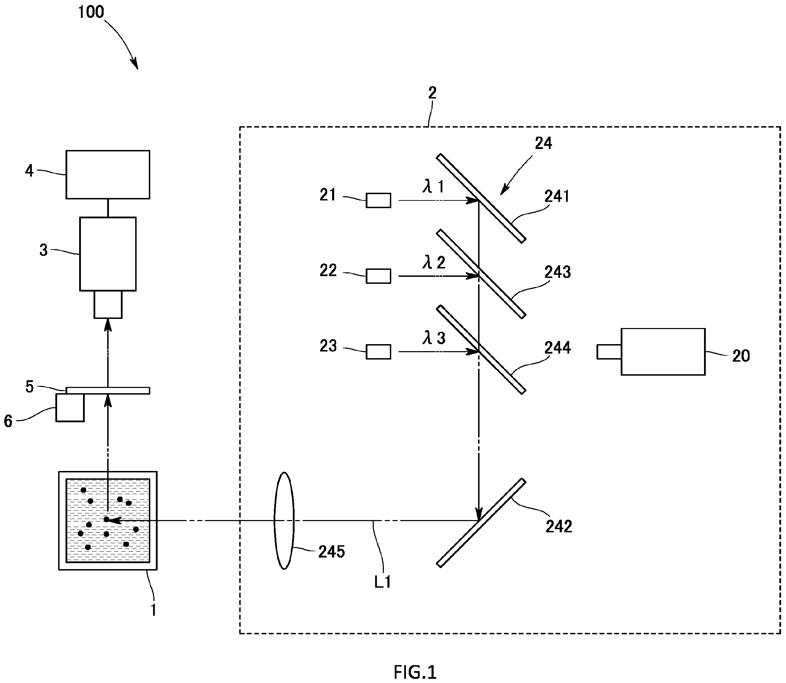

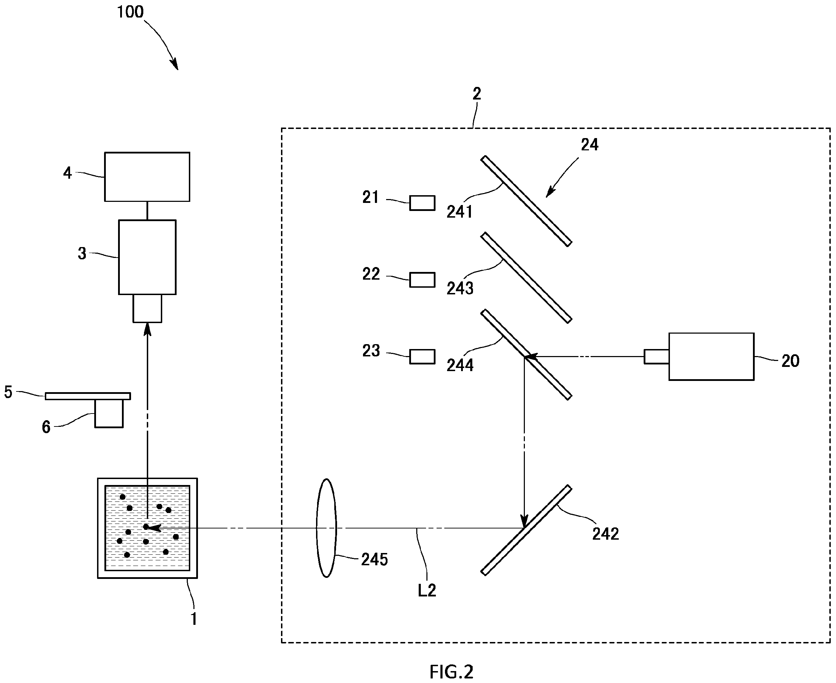

As shown in Fig. 1 and Fig. 2, the particle analysis device 100 of this embodiment analyzes physical properties of a particle contained in a sample in a cell 1, such as, for example, an exosome contained in a body fluid sample such as blood or urine, or a virus-like particle related to a vaccine, a liposome or a protein.

The particle analysis device 100 is used in conjunction with a multiple staining method. Concretely, the particle analysis device 100 analyzes the physical properties of the particle by irradiating light on a sample wherein multiple types of fluorescent markers are added to the particle, and detecting fluorescence emitted by the fluorescent markers.

However, in the analysis using this particle analysis device 100, in case that the particle contained in the sample emits the fluorescence by itself such that the particle has auto-fluorescence as a property, the fluorescent marker is not necessarily used, and the fluorescence emitted by the particle itself may be detected. For example, inorganic materials such as fluorescent beads may be used as such a particle.

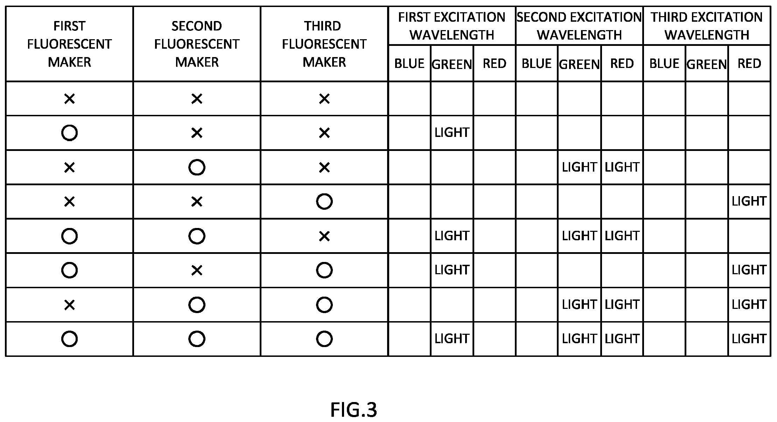

In this embodiment, as shown in Fig. 3, three types of the fluorescent markers are used. Win this configuration, it becomes possible to classify the particle to be measured into eight types. The eight types consist of one type to which all three fluorescent markers are not added, three types to which only one type is added, three types to which only two types are added, and one type to which all three types are added.

However, the particle analysis device 100 does not necessarily need to be used with the multiple staining method, however, may be used with a single type of the fluorescent marker.

The particle analysis device 100 of this embodiment can take a fluorescence observation mode described in Fig. 1, in which fluorescence produced by irradiating the fluorescent marker added to the particle with excitation light is imaged, and the scattered light observation mode described in Fig. 2, in which scattered light produced by irradiating the particle with light that is different from the excitation light is imaged.

Concretely, as shown in Fig. 1 and Fig. 2, the particle analysis device 100 comprises a light irradiation unit 2 that irradiates a sample containing the particle to be analyzed with the light, an imaging unit 3 that images the light produced by the irradiation of the light, and an information processing unit 4 that analyzes the properties of the particle using image data obtained by the imaging unit 3.

The light irradiation unit 2 has light sources 21, 22, and 23 for excitation which excite the fluorescence by irradiating the fluorescent markers with the excitation light, and a light source 20 for scattering which scatters the light other than the excitation light by irradiating the particle with the light other than the excitation light.

The light sources 21, 22, and 23 for excitation emit the light having lower intensity than that of the light source 20 for scattering, to be described later, and are, for example, LEDs. Since three types of the fluorescent markers are used in this embodiment, there are three light sources 21, 22, and 23 for excitation. One light source 21 for excitation may be used to emit the light containing three excitation wavelengths. In addition, the number of the
s 21, 22, and 23 may be appropriately changed according to the number of the used fluorescent marker types.

The first light source 21 for excitation irradiates the light with the excitation wavelength λ1 of the first fluorescent marker, the second light source 22 for excitation irradiates the light with the excitation wavelength λ2 of the second fluorescent marker, and the third light source 23 for excitation irradiates the light with the excitation wavelength λ3 of the third fluorescent marker. In this embodiment, the excitation wavelengths λ1, λ2, and λ3 are different from the fluorescence wavelengths λ1', λ2', and λ3' of the fluorescence emitted by the three fluorescent markers respectively. In addition, the light emitted from each of these light sources 21, 22, 23 for excitation is guided to the cell 1 through an irradiation optical system 24 such as reflective mirrors 241, 242, half mirrors 243, 244, and focusing lens 245.

The light source 20 for scattering irradiates the particle with the light having a wavelength different from the above-mentioned excitation wavelengths λ1, λ2, and λ3, and in this embodiment is a laser light source that emits a laser beam.

A single light source 20 for scattering is provided in this embodiment, however, a plurality of the light sources 20 for scattering may be provided.

In this embodiment, an optical path L1 (refer to Fig. 1) of the excitation light emitted from the light sources 21, 22, and 23 for excitation to the particle and an optical path L2 (refer to Fig. 2) of the laser light emitted from the light source 20 for scattering to the particle partly share. In other words, in each of the fluorescence observation mode and the scattered light observation mode, a part of the optical system consisting of the optical paths L1 and L2 is used for both modes, and in this embodiment. In this embodiment, a half-mirror 244 and a reflection mirror 242 are used for both of the optical path L1 of the excitation light and the optical path L2 of the laser light.

The imaging unit 3 images the particle to which the fluorescent marker is added by imaging the fluorescence emitted by the fluorescent marker in the fluorescence observation mode, and images various particles contained in the sample by imaging the scattered light in the scattered light observation mode regardless of whether the fluorescent marker is added or not. The imaging unit 3 outputs, for example, an image data as an image data.

The imaging unit 3 has a color discrimination capability, and in this embodiment, the imaging unit 3 is an imaging camera having a color CCD. In Fig. 1, the light irradiation direction of the light irradiation unit 2 and the imaging direction of the imaging unit 3 are orthogonal to each other, however, it is not limited to this.

In the fluorescence observation mode, if the excitation light is irradiated to the particle in the cell 1, the excitation light is scattered by the particle and the scattered light is detected by the imaging unit 3, which may interfere with fluorescence observation.

Therefore, in order to prevent the above-mentioned scattered light from being detected by the imaging unit 3 in the fluorescence observation mode, the particle analysis device 100 of this embodiment comprises one or more filters 5, as shown in Fig. 1, that cut the scattered light generated by the irradiation of the excitation light to the particle while transmitting the fluorescence in multiple colors emitted by the above-mentioned multiple types of the fluorescent markers. In case that the particle itself emits the fluorescence, the filter 5 cuts the scattered light while transmitting the fluorescence emitted by the particle.

In this embodiment, a filter group 5 (hereinafter simply referred to as the filter 5) consisting of a plurality of filters stacked on top of each other is provided between the cell 1 and the imaging unit 3. However, it is not necessary to use multiple filters. For example, in case that only one type of fluorescent marker is used, one filter may be used.

The filter 5 in this embodiment is at least a superposition of multiple notch filters, consisting of a first filter that transmits the wavelength region longer than the shortest excitation wavelength λ1, a second filter that cuts the next shortest excitation wavelength λ2, and a third filter that cuts the longest excitation wavelength λ3.

In the fluorescence observation mode, the filter 5 transmits the fluorescence of various wavelengths (various colors) while cutting at least the excitation wavelengths λ1, λ2, and λ3.

On the other hand, since it is necessary to guide the scattered light scattered by the particle into the imaging unit 3 in the scattered light observation mode, as shown in Fig. 1 and Fig. 2, the particle analysis device 100 of this embodiment is so configured that the filter 5 is arranged in front of the imaging unit 3 in the fluorescence observation mode and the filter 5 is removed from in front of the imaging unit 3 in the scattered light observation mode.

The particle analysis device 100 of this embodiment comprises a mode switching mechanism 6 that removes the filter 5 that has been arranged in front of the imaging unit 3 at a time of starting the fluorescence observation mode from in front of the imaging unit 3 after a predetermined time elapses from the time of starting the fluorescence observation mode, in order to automate the mode switching.

One example of the mode switching mechanism 6 is, for example, a mode which is configured using a motor or the like and in which the filter 5 is moved back and forth between the imaging unit 3 and the cell 1.

For example, in order to make it possible to switch the mode at a desired timing, the filter 5 may be manually arranged between the cell 1 and the imaging unit 3 by a user and manually removed from between the cell 1 and the imaging unit 3 by the user.

In accordance with the above-mentioned configuration, it is possible to observe the fluorescence generated by the use of the light sources 21, 22, and 23 for excitation in the fluorescence observation mode, and to observe the scattered light generated by the user of the scattering light source 20 in the scattered light observation mode.

The information processing unit 4 is a computer comprising a CPU, a memory, a display, and various input/output devices, and is wired or wirelessly connected to the imaging unit 3.

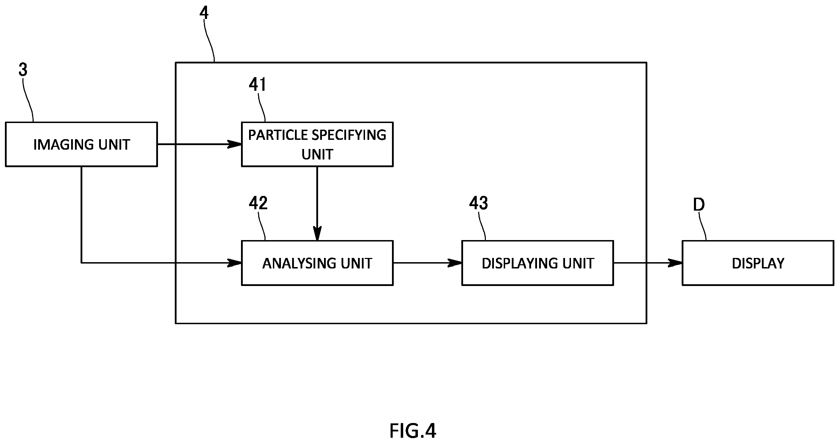

The information processing device 4 has the functions as a particle specifying unit 41, an analyzing unit 42, and a displaying unit 43, as shown in Fig. 4 by executing the program of the particle analysis device stored in the memory.

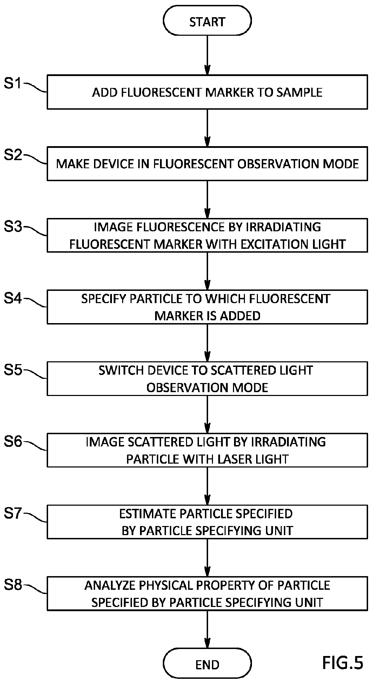

The functions of each unit of the information processing device 4 will be explained with reference to a flow chart in Fig. 5, which also serves as an explanation of the operation of the particle analysis device 100 of this embodiment.

First, the particle to be measured in the sample is multiply stained by adding multiple types of the fluorescent markers to the sample in the cell 1 (S1). As mentioned above, multiple staining is not necessarily required, and a single type of the fluorescent marker may be added to the sample. In addition, a process of agitating the sample before adding the fluorescent marker may be provided.

Next, the particle analysis device 100 is put into the fluorescence observation mode shown in Fig. 1 by arranging the filter 5 in front of the imaging unit 3 by, for example, controlling or operating the mode switching mechanism 6 (S2).

The sample in the cell 1 is then irradiated from the light sources 21, 22, and 23 for excitation with the excitation light having wavelengths λ1, λ2, and λ3 corresponding to each of the plurality types of the fluorescent markers, and the fluorescence generated thereby is imaged by the imaging unit 3 (S3).

In this embodiment, as mentioned above, the excitation light of the excitation wavelength λ1, the excitation light of the excitation wavelength λ2, and the excitation light of the excitation wavelength λ3 are irradiated in turn. By setting the switching timing of the excitation light and the frame length of the imaging unit 3 appropriately, the image data obtained by the imaging unit 3 in the fluorescence observation mode is divided into three frames: the first frame is obtained by irradiating the excitation light of the first excitation wavelength λ1, the second frame is obtained by irradiating the excitation light of the second excitation wavelength λ2, the third frame is obtained by irradiating the the excitation light of the third excitation wavelength λ3.

However, the switching timing of the excitation light and the length of the frames of the imaging unit 3 are not limited to the above-mentioned, however, the first group of the image data consisting of multiple frames may be obtained by irradiating the excitation light of the first excitation wavelength λ1, the subsequent second group of the image data consisting of multiple frames may be obtained by irradiating the excitation light of the second excitation wavelength λ2, and the further subsequent third group of image data consisting of multiple frames may be obtained by irradiating the excitation light of the third excitation wavelength λ3.

When the sample is irradiated with the excitation light of the excitation wavelength λ1, the excitation light of the excitation wavelength λ2, and the excitation light of the excitation wavelength λ3 in turn, the particle specifying unit 41 specifies the particle to which the fluorescent marker is added based on the image data of the fluorescence obtained by the imaging unit 3 (S4). When the particle itself emits the fluorescence, the particle specifying unit 41 specifies the particle that emits the fluorescence.

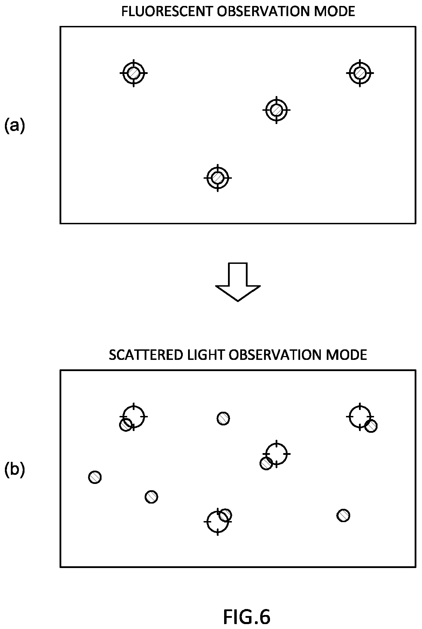

In this embodiment, what is indicated by the diagonal lines in Fig. 6(a) is the particle that is captured in the image obtained by irradiating the excitation light of the first excitation wavelength λ1 (more specifically, the image of the first frame). In other words, this image shows four particles to which the luminescent markers emitted by the excitation light of the first excitation wavelength λ1 have been added, and no other particle is shown in this image.

The particle specifying unit 41 of this embodiment specifies the position, the size, or the range of the particle shown in the image data of this fluorescence.

It is preferable that the particle specifying unit 41 specifies the particle to which the fluorescent marker is added from at least one of the image data of the fluorescence obtained in the fluorescence observation mode, for example, from the first frame to the tenth frame, and more preferably, the particle specifying unit 41 specifies the particle to which the fluorescent marker has been added from the image data of at least the first frame.

Concretely, the particle specifying unit 41 specifies the position of the particle by distinguishing the particle to which the fluorescent maker emitting the fluorescent is added from other area by image-processing the image data, and in this embodiment, the particle specifying unit 41 calculates the position of the center of the gravity of each particle to which the fluorescent marker has been added.

In the same way that the particle is specified from the image data of the first frame, the particle specifying unit 41 calculates the position of the particle (in this embodiment, the position of the center of the gravity) that is captured in the respective image data from the image data of the second and third frames.

The particle specifying unit 41 of this embodiment is configured to specify not only the position of the particle but also the type of the particle based on the respective image data.

To facilitate understanding, the particle shown in the first frame is referred to as the particle of the first type, the particle in the second frame as the particle of the second type, and the particle in the third frame as the particle of the third type.

Next, the particle analysis device 100 is switched from the fluorescence observation mode shown in Fig. 1 to the scattered light observation mode shown in Fig. 2 by removing the filter 5 from in front of the imaging unit 3 by controlling or operating the mode switching mechanism 6 (S5).

Subsequently, a laser beam is irradiated from the light source 20 for scattering to the particle in the cell 1, and the scattered light scattered by the particle is imaged by the imaging unit 3 (S6).

The scattered light observation mode is a mode wherein the number of the frame is larger than that of the fluorescence observation mode, in other words, the number of the frame (more specifically, the number of the image data) obtained by the imaging unit 3 in the scattered light observation mode is larger than the number of the frame (more specifically, the number of the image data) obtained by the imaging unit 3 in the fluorescence observation mode. In addition, in this embodiment, the observation time of the scattered light in the scattered light observation mode is longer than the observation time of the fluorescence in the fluorescence observation mode.

The analyzing unit 42 obtains the image data of the scattered light captured by the imaging unit 3 in the scattered light observation mode and analyzes the physical properties of the particle based on the image data. In addition to the image data obtained in the scattered light observation mode, the analyzing unit 42 may also analyze the physical properties of the particle using the image data obtained in the fluorescence observation mode.

Concretely, the analyzing unit 42 calculates the particle diameter distribution by the PTA method and calculates the particle diameter distribution of the particle specified by the particle specifying unit 41 by obtaining the diffusion velocity by the Brownian motion of the particle. The analyzing unit 42 does not necessarily need to calculate the particle diameter distribution, however, the analyzing unit 42 may calculate the particle diameter of the particle as a physical property.

Since the time period from the start of the fluorescence observation mode until the fluorescence observation mode is switched to the scattered light observation mode is approximately a period while three frames of the image data are obtained, and the time period is very short, there should be almost no movement of the particle during the fluorescence observation mode.

Therefore, although the number of the observed particle increases at a time when the fluorescence observation mode is switched to the scattered light observation mode, as shown in Fig. 6(b), the position of the particle specified by the particle specifying unit 41 in the fluorescence observation mode and the position at which the particle is observed in the scattered light observation mode should hardly change.

Then, the analyzing unit 42 of this embodiment estimates the particle specified by the above-mentioned particle specifying unit 41 among the particles that are shown on the scattered light image data (S7) and analyzes the estimated particle by regarding the particle as the particle specified by the particle specifying unit 41 (S8).

Concretely, the analyzing unit 42 obtains the position of the center of the gravity of each of the particles calculated by the above-mentioned particle specifying unit 41 based on the image data of the fluorescence, and also estimates the particle that is shown on the image data of the scattered light at the position that is the closest to the obtained center of the gravity position as the particle specified by the particle specifying unit 41.

The analyzing unit 42 of the present embodiment is configured to estimate each of the first, second, and third types of the particles specified by the particle specifying unit 41 from among the particles shown in the image data of the scattered light.

In accordance with this configuration, the analyzing unit 42 is configured to analyze the physical properties of the particle contained in the sample by isolating the physical properties of each type of the particles, and concretely, to calculate the particle diameter distribution of each of the particle of the first type, the particle of the second type, and the particle of the third type.

Subsequently, the displaying unit 43 displays the result of the analysis of the analyzing unit 42 on a display (D) or the like, and concretely, the displaying unit 43 displays the particle diameter distribution or the particle diameter, for example, in a manner of being specifiable for each type of the particle.

### <Effects of this embodiment>

In accordance with the particle analysis device 100 of this embodiment having the above configuration, since the particle to which the fluorescent marker is added is specified in the fluorescence observation mode and the specified particle is observed and analyzed in the scattered light observation mode, it is possible to reduce the time required for the fluorescence observation mode to a short period, and it is possible to secure a sufficient observation time by the PTA method without being affected by the fading of the color of the fluorescent marker in the subsequent scattered light observation mode.

In addition, since the filter 5 is arranged in front of the imaging unit 3 in the fluorescence observation mode and the filter 5 is removed from in front of the imaging unit 3 in the scattered light imaging mode, it is possible to use a common imaging unit 3 in both the fluorescence observation mode and the scattered light observation mode, which reduces the number of components and manufacturing costs and downsizes the particle analysis device 100.

Furthermore, since the particle analysis device 100 switches to the fluorescence observation mode or the scattered light observation mode by moving the filter 5 with the mode switching unit, it becomes possible to switch the mode, which consequently enables automating the analysis.

Moreover, since the particle specifying unit 41 specifies the particle to which the fluorescent marker is added from the image data of at least the first frame of the fluorescence obtained in the fluorescence observation mode, it is possible to shorten the time required for the fluorescence observation mode as much as possible.

In addition, analysis by the PTA method is secured by making the light sources 21, 22, 23 for excitation different from the light source 20 for scattering, and using the laser light source as the light source for scattering used in the scattered light observation mode, and by using a relatively inexpensive LED as the light source for excitation used in the fluorescence observation mode, it becomes possible to irradiate the excitation light with an excitation wavelength suitable for each of various fluorescent markers with an inexpensive device configuration by using of a relatively inexpensive LED as the light source for excitation used in the fluorescence observation mode.

Furthermore, since the particle analysis device 100 has a plurality of the light sources 21, 22, 23 for excitation that emit the excitation light of a mutually different excitation wavelength, it is possible to irradiate the excitation light of the excitation wavelength suitable for each of various fluorescent markers. This allows the particle analysis device 100 to contribute to the multiple staining method with an inexpensive configuration.

### < Other Embodiments>

The present invention is not limited to the above-mentioned embodiments.

For example, the LED is used in the above-mentioned embodiment as the light sources 21, 22, and 23 for excitation used in the fluorescence observation mode, however, the light sources that emit the light with a wavelength broader than that of the LED, such as, for example, a lamp that emits white light may also be used.

In case that multiple types of the fluorescent markers are used in such a configuration, it is preferable to provide a filter that transmits the excitation wavelength of the fluorescent marker on the optical path from the light source to the cell 1 in order to extract the excitation wavelength of each fluorescent marker out of the broad light emitted from the light source.

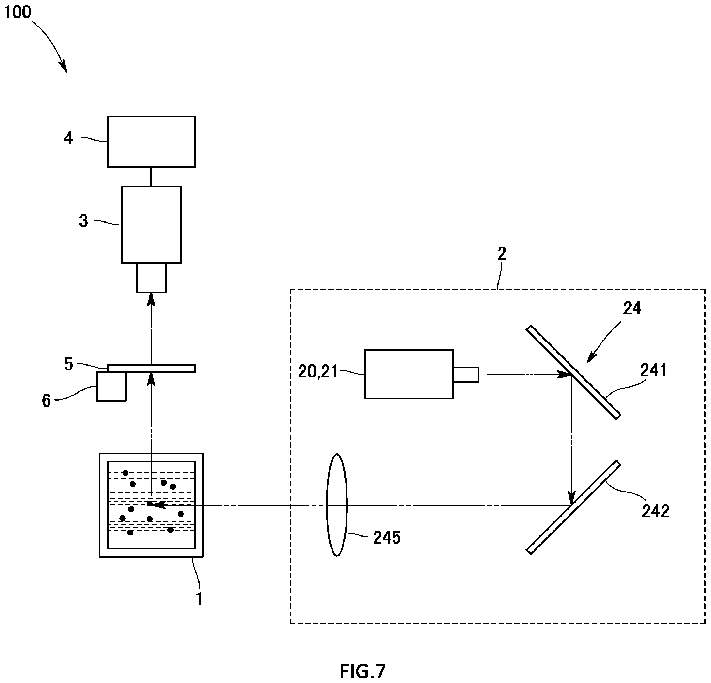

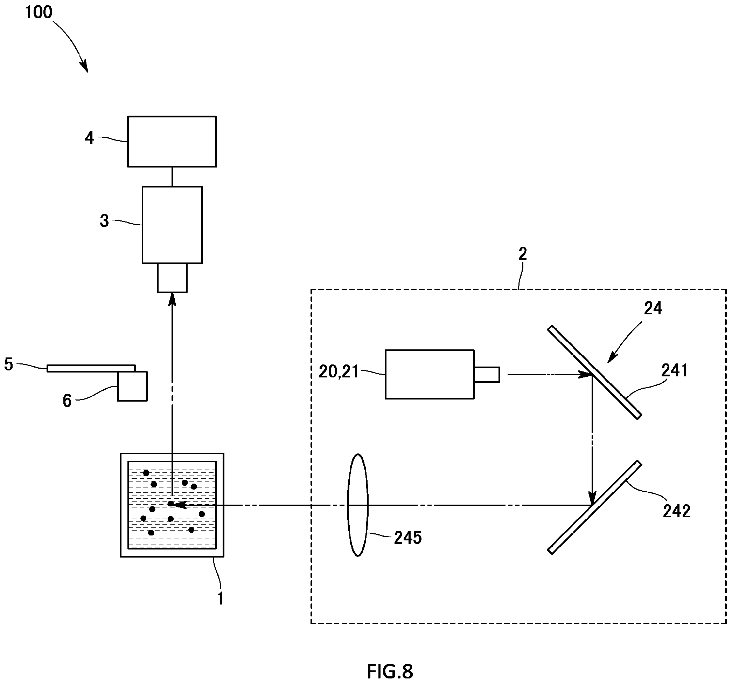

In addition, as shown in Fig. 7 and Fig. 8, the same light source may be used as both the light source 21 for excitation and the light source 20 for scattering. More concretely, the light source used as the light source 21 for excitation and the light source used as the light source 20 for scattering emit the light of the excitation wavelength for the fluorescent marker, and the light source is, for example, a laser light source.

Similar to the above-mentioned embodiment, the particle analysis device 100 of this embodiment comprises a filter 5 that cuts the excitation light emitted from the laser light sources 20 and 21 respectively, while transmitting the fluorescence emitted by the fluorescent marker.

In accordance with this configuration, the filter 5 is placed between the cell 1 and the imaging unit 3 at the start of the fluorescence observation mode as shown in Fig. 7, and the particle specifying unit 41 specifies the particle to which the fluorescent marker is added from the image data obtained by the imaging unit 3.

After a predetermined time has elapsed from the start of the fluorescence observation mode, the scattered light observation mode is set by removing the filter 5 from between the cell 1 and the imaging unit 3 as shown in Fig. 8, and the analyzing unit 42 analyzes the physical properties of the particle specified by the particle specifying unit 41.

Even with this configuration, since the particle to which the fluorescent marker is added is specified in the fluorescence observation mode, and the specified particle is observed and analyzed in the scattered light observation mode, the time required as the fluorescence observation mode can be reduced to be a short period. This makes it possible to secure sufficient observation time using the PTA method without being affected by the fading of the fluorescent markers.

In addition, in the above-mentioned various embodiments, in order to extend the time until fading occurs as much as possible, it is preferable to make the analysis conditions different between the fluorescence observation mode and the scattered light observation mode, and it is preferable that the analysis condition at least such as one of the excitation light intensity, the gain or the exposure time of the imaging unit 3 and the filter position is different.

As a more concrete embodiment represented are that the intensity of the excitation light (the output of the laser light source) in the fluorescence observation mode is made lower than that in the scattered light observation mode, the gain of the imaging unit 3 is made larger, the exposure time of the imaging unit 3 is made longer, or a light reducing filter is arranged between the cell 1 and the light sources 21, 22, 23 for excitation in the fluorescence observation mode. A combination of these two or more modes is more preferable. In the scattered light observation mode, it is preferable that the light reducing filter is removed from between the cell 1 and the light sources 21, 22, 23 for excitation.

Furthermore, in case of switching the particle analysis device 100 from the fluorescence observation mode to the scattered light observation mode, the removal of the filter 5 from in front of the imaging unit 3 may increase the amount of the light observed in the scattered light observation mode, resulting in white skipping. In the scattered light observation mode, a light-reducing filter may be placed between the cell 1 and the imaging unit 3 in the scattered light observation mode.

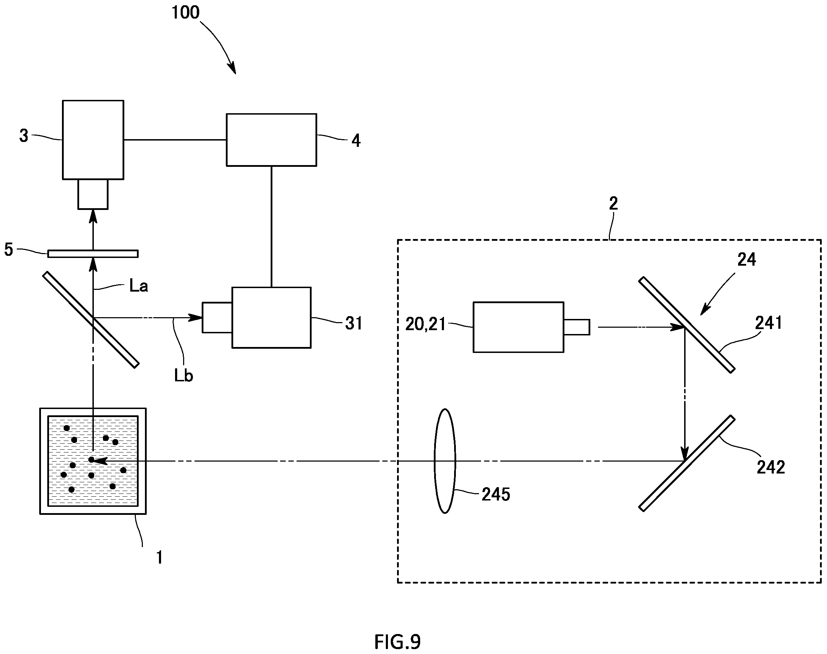

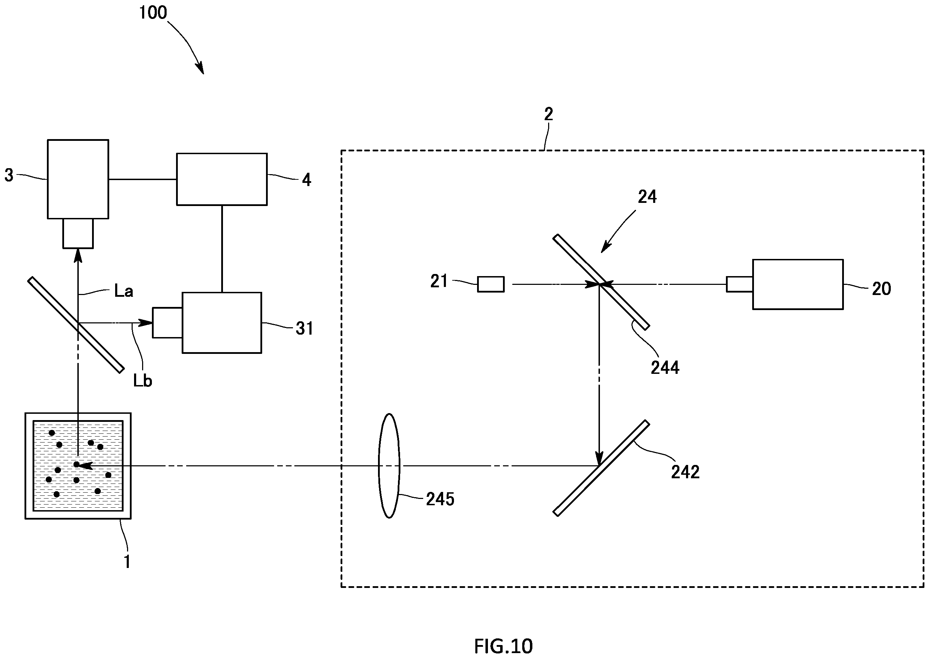

A mode in which the fluorescence observation mode and the scattered light observation mode are switched by moving the filter 5 has been explained, however, as shown in Fig. 9 and Fig. 10, the particle analysis device 100 may be configured to be switched between the fluorescence observation mode and the scattered light observation mode while the filter 5 remains in place.

As shown in Fig. 9, a configuration of the particle analysis device 100 is represented by that further comprising a beam splitter that splits the light flux consisting of the fluorescence and the scattered light passing through the cell 1 into a first optical path (La) and a second optical path (Lb), and the imaging unit 3 for observing the fluorescence and the filter 5 are arranged on the first optical path (La) similar to the above-mentioned embodiment, and a second imaging unit 31 that observes the scattered light is arranged separately from the imaging unit 3 on the second optical path (Lb).

In other words, the first optical path (La) is an optical path for observing the fluorescence and the second optical path (Lb) is an optical path for observing the scattered light, and these optical paths (La) (Lb) are formed independently of each other so that the fluorescence and the scattered light can be observed simultaneously.

In the fluorescence observation mode during the initial analysis time, the particle specifying unit 41 specifies the particle to which the fluorescent marker is added from the image data obtained by the imaging unit 3, and the analyzing unit 42 analyzes the physical properties of the particle specified by the particle specifying unit 41 from the image data obtained by the second imaging unit 31 by switching the particle analysis device 100 from the fluorescence observing mode to the scattered light observation mode at a time of a subsequent analysis.

Even with this configuration, it is possible to make the time required as the fluorescence observation mode short to a small amount. And it is possible to make the mechanism and operation to remove the filter 5 from in front of the imaging unit 3 unnecessary by placing the filter 5 on the first optical path (La), and furthermore, it is possible to observe the fluorescence and the scattered light simultaneously.

Furthermore, if the excitation wavelength of the fluorescent marker and the wavelength of the fluorescence are different and the imaging unit 3 and the second imaging unit 31 have a color CCD having a color discrimination function, the particle analysis device 100 need not necessarily have a filter 5 to transmit the fluorescence, as shown in Fig. 10. Then the fluorescence can be observed by the imaging unit 3 and the scattered light can be observed by the second imaging unit 31.

The imaging unit 3 need only have an ability to discriminate a color. For example, the imaging unit 3 may be configured with multiple sets of a combination of a filter 5 that transmits the fluorescence of a certain color and a CCD camera.

In addition, the imaging unit 3 may make use of a monochrome CCD camera that does not have the ability to discriminate a color.

Furthermore, although the excitation light from the three light sources 21, 22, and 23 for excitation is irradiated in sequence in the above embodiment, if the imaging unit 3 has a color discrimination function as described above, the excitation light from multiple light sources for excitation may be irradiated simultaneously, for example, in the first frame.

In accordance with this configuration, the particle specifying unit 41 can specify each of the multiple types of the particles by the use of a common image data, for example, in the first frame, and the fluorescence observation mode can be made shorter.

In addition, the particle analysis device 100 of the present invention may be configured that the particle is specified in the fluorescence observation mode and the physical properties is analyzed in the scattered light observation mode, and then the particle in the cell 1 are automatically or manually agitated, followed by specifying the particle in the fluorescence observation mode and analyzing again the physical properties in the scattered light observation mode.

Furthermore, the analyzing unit 42 is not limited to measuring the particle size distribution or the particle diameter, however, the analyzing unit 42 may also analyze various properties of the particle such as the number concentration of the particle and the gel properties such as the average lattice spacing of the gel from the autocorrelation function.

It goes without saying that the present invention is not limited to the above-mentioned embodiments, and various modifications are possible without departing from a spirit of the invention.

### Possible applications in industry

In accordance with the present claimed invention, it is possible to secure an enough time for observation by the PTA method without being affected by fading of the particle having characteristics of the fluorescent marker or an autofluorescence.

### Explanation of codes

- 100 ...: particle analysis device
- 1 ...: cell
- 2 ...: light irradiation unit
- 3 ...: imaging unit
- 4 ...: information processing unit
- 41 ...: particle specifying unit
- 42 ...: analyzing unit
- 43 ...: displaying unit
- 5 ...: filter
- 6 ...: mode switching mechanism

## Claims

1. A particle analysis device that can take a fluorescence observation mode in which fluorescence emitted by a fluorescent marker added to a particle or by the particle itself is imaged by irradiating the particle with excitation light, and a scattered light observation mode in which scattered light produced by irradiating the particle with the light is imaged, wherein comprising
a particle specifying unit that specifies the particle to which the fluorescent marker is added or the particle that emits the fluorescence from fluorescence image data obtained in the fluorescence observation mode, and
an analyzing unit that analyzes physical properties of the particle by obtaining diffusion speed due to the Brownian motion of the particle specified by the particle specifying unit from the image data of the scattered light obtained in the scattered light observation mode having a frame number larger than that in the fluorescence observation mode.

2. The particle analysis device described in claim 1, wherein comprising
a filter that cuts the scattered light produced by irradiating the particle with the excitation light while transmitting the fluorescence emitted by the fluorescent marker or the particle itself, and
an imaging unit that images the fluorescence transmitted through the filter.

3. The particle analysis device described in claim 2, wherein the filter is arranged in front of the imaging unit in the fluorescence observation mode, and the filter is removed from in front of the imaging unit in the scattered light observation mode.

4. The particle analysis device described in claim 3, further comprising a mode switching mechanism that removes the filter that has been arranged in front of the imaging unit at a time of starting the fluorescence observation mode from in front of the imaging unit after a predetermined time elapses from the start of the fluorescence observation mode.

5. The particle analysis device described in claim 2, further comprising a beam splitter that splits a luminous flux comprising the fluorescence and the scattered light into a first optical path and a second optical path, wherein
the imaging unit and the filter are arranged on the first optical path, and
a second imaging unit that is different from the imaging unit that observes the scattered light is arranged on the second optical path.

6. The particle analysis device described in either one of claim 1 to claim 5, wherein the particle specifying unit specifies the particle to which the fluorescent marker is added or the particle that emits the fluorescence from at least the first frame of the image data of the fluorescence obtained in the fluorescence observation mode.

7. The particle analysis device described in either one of claim 1 to claim 6, wherein at least one of intensity of the excitation light, a gain of the imaging unit, exposure time of the imaging unit and a position of the filter differs between the fluorescence observation mode and the scattered light observation mode.

8. A particle analysis device program for use in a particle analysis device that can take a fluorescence observation mode in which fluorescence emitted by a fluorescent marker added to a particle or by the particle itself is imaged by irradiating the particle with excitation light, and a scattered light observation mode in which scattered light produced by irradiating the particle with the light is imaged, wherein making a computer produce functions as a particle specifying unit that specifies the particle to which the fluorescent marker is added or the particle that emits the fluorescence from fluorescence image data obtained in the fluorescence observation mode, and an analyzing unit that analyzes physical properties of the particle by obtaining diffusion speed due to the Brownian motion of the particle specified by the particle specifying unit from the image data of the scattered light obtained in the scattered light observation mode having a frame number larger than that in the fluorescence observation mode.

9. A particle analysis method using a particle analysis device that can take a fluorescence observation mode in which fluorescence emitted by a fluorescent marker added to a particle or by the particle itself is imaged by irradiating the particle with excitation light, and a scattered light observation mode in which scattered light produced by irradiating the particle with the light is imaged, wherein
the particle to which the fluorescent marker is added or the particle that emits the fluorescence from fluorescence image data obtained in the fluorescence observation mode is specified, and
the physical properties of the particle by obtaining diffusion speed due to the Brownian motion of the particle specified by the particle specifying unit from the image data of the scattered light obtained in the scattered light observation mode having a frame number larger than that in the fluorescence observation mode is analyzed.
